# FASCICULE DE BREVET EUROPEEN

(11) **EP 1 051 086 B1**
(45) Date de publication et mention de la délivrance du brevet: **17.04.2002**
(21) Numéro de dépôt: 99900957.4
(22) Date de dépôt: 20.01.1999
(51) Int. Cl.: A23L 1/237, A23L 1/304, A61K 33/00

(54) **COMPOSITION DIETETIQUE SOUS FORME D'UN SEL DE SUBSTITUTION DU SEL DE TABLE**
DIÄTETISCHE ZUSAMMENSETZUNG IN FORM EINES TISCHSALZERSATZES
DIETETIC COMPOSITION IN THE FORM OF A SUBSTITUTION SALT FOR TABLE SALT

(30) Priorité: 26.01.1998 FR 9800800
(43) Date de publication de la demande: 15.11.2000
(73) Titulaire: SANOFI-SYNTHELABO, 75013 Paris (FR)
(72) Inventeur: DERRIEN, Marcel, F-78150 Rambouillet (FR); FONTVIEILLE, Anne-Marie, F-78110 Le Vésinet (FR)
(74) Mandataire: Thouret-Lemaitre, Elisabeth
(86) Numéro de dépôt international: FR9900098
(87) Numéro de publication internationale: WO9937170

(56) Documents cités:
- EP-A- 0 291 578
- EP-A- 0 636 321
- DE-A- 2 305 980
- GB-A- 2 237 720
- US-A- 4 107 346

## Description

La présente invention se rapporte, d'une manière générale, à une composition diététique sous forme d'un sel de substitution du sel de table ordinaire.

En particulier, l'invention concerne une composition diététique sous forme de sel de substitution appauvri en sodium, utile comme complément en cas d'hypertension artérielle légère ou modérée.

Le bénéfice d'une correction pharmacologique de l'hypertension artérielle sur les risques de complications cardio-vasculaires a été clairement démontré.
Ainsi, une baisse de pression artérielle de 6mm Hg entraîne une réduction du risque des accidents vasculaires cérébraux et de l'infarctus du myocarde de respectivement 42 et 14%.

Pour cette raison, on recommande généralement une approche non pharmacologique dans le cas d'hypertension artérielle du moins dans la phase initiale de la prise en charge d'une hypertension légère voire modérée, ou en association avec un traitement médicamenteux.

Cette attitude est motivée par l'abondante littérature qui montre un lien statistique ou même physiopathologique existant entre certains facteurs et l'hypertension artérielle. Ainsi, le tabagisme, la consommation excessive d'alcool, le surpoids, certains déséquilibres alimentaires concernant les apports en sodium, potassium, calcium, magnésium, le stress et la sédentarité sont incriminés comme facteurs ou cofacteurs dans la survenue, le maintien ou l'aggravation d'une hypertension.

Les résultats de nombreuses études tendent à montrer le rôle important des sels minéraux dans la régulation de la pression artérielle : le sodium augmenterait cette pression alors que l'inverse semble démontré pour le potassium et le magnésium.

En effet, des études épidémiologiques suggèrent une relation inverse entre l'apport alimentaire de l'ion K⁺ et la prévalence de l'hypertension artérielle.

Une méta-analyse publiée dans JAMA : 1997; 277 : 1624 - 1632 et portant sur 33 études rapporte, d'ailleurs, que la supplémentation orale en potassium est associée à une réduction de 3,11 mm Hg pour la pression systolique et de 1,97 mm Hg pour la pression diastolique. L'effet, hypotenseur semblait en outre plus marqué chez les sujets qui ont une consommation plus élevée en sodium.

Ainsi, il semble démontré qu'un apport supplémentaire en ion K⁺ pourrait diminuer la pression artérielle.

Dans les populations habituées à une alimentation riche en potassium l'incidence d'accidents vasculaires cérébraux paraît d'ailleurs être réduite.

De plus, il a été montré qu'une déplétion provoquée en potassium, de courte durée, aggrave une hypertension préexistante et induit une augmentation de la pression artérielle chez des volontaires normo-tendus. Parmi les nombreux mécanismes invoqués, le plus important semble être l'effet natriurétique de l'ion K⁺, ce qui expliquerait la mauvaise réponse obtenue lors d'apports supplémentaires en potassium chez des personnes sous restriction sodée.

De nombreuses études ont montré par ailleurs une relation plus importante du rapport Na⁺/K⁺ avec la pression artérielle qu'avec Na⁺ ou K⁺ seuls. Il en résulte que les mesures diététiques pour réduire la pression artérielle semblent plus efficaces lorsque les apports en différents sels minéraux sont changés simultanément.

Ainsi, à la suite d'études interventionnelles, il paraît raisonnable d'encourager une alimentation riche en potassium ou d'avoir recours à une supplémentation.

D'autre part, un apport supplémentaire en magnésium est également recommandé chez l'hypertendu. Cette recommandation se base sur des observations qui ont été réalisées au départ chez le rat où un déficit en magnésium entraîne l'apparition d'une hypertension marquée.

De plus, l'ion Mg⁺⁺, un vasodilatateur antagoniste naturel de l'ion Ca⁺⁺ à de nombreux niveaux, constitue un cofacteur de nombreux enzymes et sa présence déficitaire pourrait produire des détériorations hémodynamiques et des arythmies ventriculaires.

Récemment, on a d'ailleurs démontré que l'ion Mg⁺⁺, administré dans la phase aiguë de l'infarctus du myocarde, réduit la survenue d'arythmies et la mortalité.

Un apport supplémentaire en magnésium est en outre indiqué lors d'hypokaliémie résistante généralement due à une hypomagnésémie. Cette supplémentation peut être tentée d'ailleurs pendant quelques semaines sans grand risque lorsqu'un déficit est mis en évidence ou est fortement suspecté.

Au surplus, l'intérêt d'un apport supplémentaire simultané en potassium et magnésium avec diminution de l'apport en sodium notamment en vue d'une diminution de la tension artérielle a été clairement démontré.

Par exemple, on a publié dans British Medical Journal 1994 ; 309 : 436 - 440, une étude conduite durant 24 semaines chez des personnes âgées présentant une hypertension modérée.

A la suite de cette étude, on a pu montrer que le remplacement du sel de table ordinaire (chlorure de sodium) par un sel comportant 41% de chlorure de sodium, 41% de chlorure de potassium, 17% de sels de magnésium et 1% de minéraux à l'état de trace provoque une réduction de 7,6 mm Hg de la pression systolique et de 3,3 mm Hg de la pression diastolique.

Ces résultats amènent à conclure qu'un sel de substitution pauvre en ion Na⁺ mais enrichi en ion K⁺ et en ion Mg⁺⁺ offre une approche non pharmacologique intéressante pour réduire une hypertension faible à modérée.

Cet essai, comme d'autres également publiés (Circulation Supplément, 1996, vol. 94, N° 8, p 1983) suggèrent, en conséquence, que la substitution du sel de table ordinaire par un sel pauvre en ion Na⁺ mais par contre riche en ions K⁺ et Mg⁺⁺ pourrait avoir un intérêt comme adjuvant thérapeutique notamment dans le traitement de l'hypertension modérée.

De nombreuses autres compositions utiles comme sels de substitution ont été proposées en vue de réduire l'apport joumalier en ion Na⁺.

Parmi celles-ci, on trouve notamment des sels de substitution apprauvris en sodium mais enrichis en potassium et magnésium en quantités substantielles.

A cet effet, on peut retenir :
a) le brevet GB 2015803 qui décrit un sel de substitution contenant, en poids de la composition, 50 à 65% de NaCI, 20 à 40% de KCl ou K₂SO₄ et 5 à 20% de MgCl₂ ou MgSO₄,
b) le brevet US 4473595 qui rapporte un sel de substitution contenant en poids de la composition, 40 à 50% de NaCI, 25 à 35% de KCI et 15 à 25% de MgCl₂ ou MgSO₄,
   D'autre part, des sels de substitution du sel de table regroupant des sels de sodium, de potassium, de magnésium et de calcium sont également connus et ont été publiés.
   Toutefois, les sels de magnésium et les sels de calcium y sont présents en quantités relativement faibles.
   On peut citer à cet effet :
c) le brevet US 4107346 où l'on décrit une composition de remplacement du sel de table qui comprend des ions Na⁺, K⁺, Mg⁺⁺, et Ca⁺⁺, en proportions correspondant substantiellement à celles présentes dans les fluides extra-cellulaires du corps humain, ces proportions comprenant en poids 92-93,1% d'ion Na⁺ ; 2,4 à 3,4% d'ion K⁺ ; 3,1 à 3,4% d'ion Ca⁺⁺ ; 1,2 à 1,4% d'ion Mg⁺⁺.
d) le brevet GB 2237720 qui cite un sel diététique formé de sel marin ou de sel gemme enrichi en KCl, c'est-à-dire de composition finale comprenant en poids 46,6% de NaCl ; 6,5% de MgCl₂ ; 2,8% de MgSO₄ ; 2,2% de CaSO₄ ; 41,5% de KCl ; 0,10% de MgBr₂ et 0,2% de CaCO₃.
e) le brevet EP 0291578 qui décrit un substitut du sel de table contenant de 40 à 85% en poids de sel gemme, 5 à 45% en poids de KCI, 2 à 10% en poids de CaCO₃ et 2 à 10% en poids du MgCO₃.

On observera toutefois que dans chacun des sels de substitution de l'état de la technique rapporté ci-dessus, le chlorure de sodium reste présent à raison d'au moins 40% en poids, c'est-à-dire en quantité relativement importante.

La recherche d'un sel de substitution du sel de table ordinaire, comprenant le chlorure de potassium, un sel de magnésium ainsi que le chlorure de sodium, lui-même en quantité pondérale proportionnellement moins importante que dans les compositions antérieures, ce sel de substitution présentant de surcroît des qualités gustatives et un pouvoir salant acceptables, reste d'un intérêt essentiel.

Or, on a trouvé de manière surprenante qu'en remplaçant partiellement, par des sels de calcium, le chlorure de sodium des sels de substitution de l'état de la technique, on peut obtenir des compositions utilisables comme compléments diététiques dans l'hypertension légère ou modérée tout en étant dotées à la fois d'une saveur assez analogue à celle du sel de table ordinaire et d'un pouvoir salant sensiblement égal voire supérieur à celui-ci.

La présente invention a donc pour objet une composition diététique, sous forme d'un sel de substitution du sel de table, comprenant en poids, de :
40% à 50% de chlorure de potassium
15% à 25% de chlorure de sodium
15% à 25% d'un ou de plusieurs sels de calcium
8% à 15% d'un ou de plusieurs sels de magnésium.

Comme sel de calcium, on utilise avantageusement un phosphate c'est-à-dire le phosphate monocalcique, le phosphate bicalcique, le phosphate tricalcique ou le glycerol-phosphate de calcium. Ce sel de calcium peut être également le di-citrate de calcium ou le D-gluconate de calcium.

Toutefois, on préfère le phosphate monocalcique c'est-à-dire Ca (H₂PO₄)₂.

De même, le sel de magnésium peut être un phosphate de magnésium, le gluconate de magnésium ou le citrate dibasique de magnésium. Ce dernier est d'ailleurs utilisé à titre préférentiel dans les compositions diététiques selon l'invention.

On a observé en outre que les sels de calcium ou de magnésium présents dans les compositions diététiques selon l'invention, notamment le phosphate monocalcique et le citrate dibasique de magnésium sont dotés de qualités gustatives généralement supérieures à celles des lactate, chlorure ou hydroxyde de calcium ou encore des chlorure ou sulfate de magnésium.

De manière à leur permettre un écoulement facile et sans formation d'agglomérats, les compositions selon l'invention contiendront, si nécessaire, un ou plusieurs agents antiagglomérants à raison de 0,5% à 2,5% en poids de la composition totale notamment de 0,5% à 1% en poids de cette composition.

Habituellement, on utilise le carbonate de magnésium comme antiagglomérant.

Toutefois, d'autres agents de ce type tels que la silice colloïdale, le silicate de magnésium, l'acide stéarique, le stéarate de magnésium ou un phosphate de calcium, peuvent être avantageusement envisagés.

Au surplus, les compositions diététiques selon l'invention peuvent contenir éventuellement un ou plusieurs agents exhausteurs de goût, à raison de 0,5% à 2,5% en poids de la composition totale notamment de 0,5% à 2% en poids de cette composition. Cet exhausteur de goût, qui contribue notamment au masquage de l'amertume de l'ion K⁺ et à l'impression de salinité, est de préférence l'acide glutamique, un glutamate tel que le glutamate de calcium ou le glutamate de magnésium, l'acide ascorbique, un ascorbate tel que l'ascorbate de calcium ou l'ascorbate de magnésium, l'acide citrique ou un citrate tel que le citrate de calcium ou le citrate de magnésium.

Si nécessaire, les compositions diététiques selon l'invention peuvent inclure des traces d'un composé iodé de préférence l'iodure de potassium pour obtenir un sel de substitution iodé. Ce composé iodé et, de préférence l'iodure de potassium, est ajouté habituellement à raison d'environ 0,01 % en poids de la composition finale.

Selon un aspect particulier et préférentiel, l'invention se rapporte à une composition diététique sous forme d'un sel de substitution du sel de table, comprenant, en poids, de :
45% à 50% de chlorure de potassium
15% à 20% de chlorure de sodium
15% à 20% d'un ou de plusieurs sels de calcium
10% à 15% d'un ou de plusieurs sels de magnésium
et éventuellement de :
0,5% à 1 % d'un ou plusieurs agents antiagglomérants
0,5% à 2% d'un ou plusieurs agents exhausteurs de goût.

Les compositions diététiques selon l'invention se sont révélées exemptes d'arrière-goût ou de saveur amère et leur utilisation comme condiment ou source d'assaisonnement procure une perception de goût analogue à celle du sel de table ou chlorure de sodium.

En outre, malgré leurs faibles teneur en chlorure de sodium, les compositions diététiques selon l'invention, par leur pouvoir salant tout à fait intéressant, pourraient réduire d'au moins 60% la consommation journalière en ion Na⁺.

Comparativement aux sels de substitution de l'état de la technique, les compositions diététiques selon l'invention se caractérisent principalement par le remplacement d'une certaine proportion pondérale de chlorure de sodium par une proportion équivalente d'un ou de plusieurs sels de calcium.

Ces sels de calcium confèrent aux compositions selon l'invention, non seulement des qualités gustatives tout à fait acceptables mais contribuent de manière significative au contrôle de l'hypertension faible ou modérée.

En effet, il est connu que l'augmentation de l'apport alimentaire en calcium réduit la pression artérielle et affecte favorablement la fonction artérielle du muscle lisse dans différentes formes d'hypertension expérimentales.

Ainsi, les compositions diététiques selon l'invention, sous forme de sels de substitution du sel de table, pourront être utilisées avantageusement en vue d'augmenter les apports en magnésium et en calcium. A ce titre, elles sont particulièrement avantageuses sur le plan nutritionnel. Ces apports sont souvent insuffisants chez les sujets qui présentent une hypertension artérielle légère ou modérée.

La présente invention a également pour objet l'utilisation d'une composition telle que définie ci-dessus ou ci-après pour la préparation d'une composition pharmaceutique utile comme adjuvant au traitement de l'hypertension artérielle légère ou gravidique, dans la prévention de l'hypertension artérielle, dans la correction des déficiences en magnésium, dans la prévention ou le traitement de la rétention hydrosodée, ou encore chez des personnes qui désirent réduire leur consommation en sel de table ordinaire. La composition revendiquée est en particulier utile pour la préparation d'une composition pharmaceutique utile pour le traitement de l'hypertension artérielle légère ou gravidique, la prévention de l'hypertension artérielle, la correction des déficiences en magnésium, et/ou la prévention où le traitement de la rétention hydrosodée.

Des analyses sensorielles ont été effectuées en vue de déterminer la valeur moyenne de concentrations iso-salées d'un sel de substitution selon l'invention de formulation en poids :

| | |
|---|---|
| chlorure de potassium | 45% |
| chlorure de sodium | 20% |
| phosphate monocalcique | 20% |
| citrate dibasique de magnésium | 12% |
| carbonate de magnésium | 1% |
| acide ascorbique | 1% |
| acide glutamique | 1% |

et ce, comparativement au sel de table. Par "concentration iso-salée", on entend la concentration du sel de substitution selon l'invention donnant la même intensité salée en bouche qu'une solution de référence de sel de table.

Ces résultats ont permis alors de calculer le pouvoir salant de ce sel de substitution représenté par le rapport entre la concentration de la solution de référence et la valeur moyenne de concentrations iso-salées déterminées lors de plusieurs essais.

### a) Concentration iso-salée dans la purée

On a utilisé à cet effet la méthode dite "up-and-down" dont l'intérêt est de permettre d'obtenir très rapidement une estimation correcte de l'intensité salante d'une solution ou d'un aliment comparativement à une solution ou à un aliment de référence comportant une concentration donnée de sel de table (NaCI).

Cette méthode a consisté à présenter, à un groupe de 27 gouteurs expérimentés et entraînés, des stimuli d'intensité croissante ou décroissante en fonction des réponses des sujets.

On a mis en oeuvre à cet effet un protocole semblable à celui d'une épreuve de classement par paire, chaque paire étant formée d'un stimulus variable (sel de substitution de l'invention) et d'une référence constante (sel de table).

Dans le test ci-dessus, on a utilisé comme référence une purée contenant 0,6g de sel de table (NaCl)/100g de purée et comme stimulus variable une gamme de 8 concentrations décroissantes du sel de substitution de l'invention, à partir d'une concentration maximale de 4g/100g de purée avec un pas de décroissance de 1,5.

Par conséquent, les concentrations de sel de substitution étaient de 4g/100g ; 2,67g/100g ; 1,77g/100g ; 1,18g/100g ; 0,79g/100g ; 0,53g/100g ; 0,35g/100g ; 0,23g/100g ; 0,16g/100g.

Pendant les épreuves, les purées ont été maintenues au chaud dans des yaourtières. En outre, les expérimentateurs ont fonctionné par binôme, un expérimentateur étant goûteur, l'autre testeur, puis on a inversé les rôles.

A chaque présentation des paires à goûter, l'expérimentateur goûteur devait apprécier laquelle des deux purées était la plus salée.

On a ainsi trouvé une valeur moyenne de concentrations iso-salées de 0,33g/100g de purée.

En d'autres termes, il a fallu en moyenne 0,33g de sel de substitution dans 100g de purée pour conférer la même intensité de saveur salée que 0,6g de sel de table dans 100g de purée.

### b) Pouvoir salant dans la purée

Le pouvoir salant mis en évidence par un sujet correspond au rapport de la concentration de référence sur la valeur moyenne de concentrations iso-salées tandis que le pouvoir salant mis en évidence par le groupe d'expérimentateurs équivaut à la moyenne des pouvoirs salants obtenue pour chaque sujet.

Dans le test ci-dessus, le pouvoir salant du sel de substitution mis en évidence par le groupe d'expérimentateurs était de 2,07.

En conclusion, le pouvoir salant du sel de substitution de l'invention dans la purée est tout à fait remarquable compte tenu de sa faible teneur en chlorure de sodium (20% en poids).

Pris dans cet aliment, le sel de substitution pourrait diminuer de 90% les apports en chlorure de sodium tout en permettant une couverture de 28% des apports quotidiens recommandés en ion Mg⁺⁺ et de 45% des apports quotidiens recommandés en ion Ca⁺⁺ pour 5g de consommation journalière.

Les compositions diététiques selon l'invention peuvent être préparées en mélangeant après calibrage les différents ingrédients entrant dans la formulation de manière à obtenir un mélange homogène exempt de ségrégation.

L'Exemple non limitatif suivant illustre la préparation d'une telle composition diététique de l'invention.

### EXEMPLE

On prépare une composition diététique de l'invention de formulation :

| | |
|---|---|
| chlorure de potassium | 45% |
| chlorure de sodium | 20% |
| phosphate monocalcique | 20% |
| citrate dibasique de magnésium | 12% |
| carbonate de magnésium | 1% |
| acide ascorbique | 1% |
| acide glutamique | 1% |

par application du procédé suivant :

On pèse l'ensemble des ingrédients entrant dans la composition et on réalise pendant 5 minutes et sous agitation (24 tours/min), un prémélange sur mélangeur par retournement. On procède alors au calibrage du prémélange sur grille de 0,8mm d'ouverture de maille et on mélange à nouveau sous agitation (24 tours/min) durant 20 minutes.

On reprend le mélange obtenu en le calibrant sur grille de 0,5mm d'ouverture de maille puis on procède au mélange final sous agitation (24 tours/min) pendant 15 minutes.

## Revendications

1. Composition diététique sous forme d'un sel de substitution du sel de table, **caractérisée en ce qu'**elle comprend, en poids, de :
40% à 50% de chlorure de potassium
15% à 25% de chlorure de sodium
15% à 25% d'un ou plusieurs sels de calcium
8% à 15% d'un ou plusieurs sels de magnésium
et éventuellement de :
0,5% à 2,5% d'un ou plusieurs agents antiagglomérants
0,5% à 2,5% d'un ou plusieurs agents exhausteurs de goût.

2. Composition diététique selon la revendication 1, **caractérisée en ce qu'**elle comprend, en poids, de :
0,5% à 1% d'un ou de plusieurs agents antiagglomérants
0,5% à 2% d'un ou de plusieurs exhausteurs de goût

3. Composition diététique selon la revendication 1 ou 2, **caractérisée en ce qu'**elle comprend, en poids, de :
45% à 50% de chlorure de potassium
15% à 20% de chlorure de sodium
15% à 20% d'un ou de plusieurs sels de calcium
10% à 15% d'un ou de plusieurs sels de magnésium
et éventuellement de :
0,5% à 1 % d'un ou plusieurs agents antiagglomérants
0,5% à 2% d'un ou plusieurs agents exhausteurs de goût.

4. Composition diététique selon l'une des revendications 1 à 3, **caractérisée en ce que** le sel de calcium est le phosphate monocalcique, le phosphate bicalcique, le phosphate tricalcique, le glycérol-phosphate de calcium, le di-citrate de calcium ou le D-gluconate de calcium.

5. Composition diététique selon l'une des revendications 1 à 4, **caractérisée en ce que** le sel de magnésium est un phosphate de magnésium, le gluconate de magnésium ou le citrate dibasique de magnésium.

6. Composition diététique selon la revendication 4, **caractérisée en ce que** le sel de calcium est le phosphate monocalcique.

7. Composition diététique selon la revendication 5, **caractérisée en ce que** le sel de magnésium est le citrate dibasique de magnésium.

8. Composition diététique selon l'une des revendications 1 à 7, **caractérisée en ce qu'**elle comprend en outre au moins un agent antiagglomérant choisi parmi le carbonate de magnésium, la silice colloïdale, le silicate de magnésium, l'acide stéarique, le stéarate de magnésium et un phosphate de calcium.

9. Composition diététique selon l'une des revendications 1 à 8, **caractérisée en ce qu'**elle comprend en outre au moins un agent exhausteur de goût choisi parmi l'acide glutamique, le glutamate de calcium, le glutamate de magnésium, l'acide ascorbique, l'ascorbate de calcium, l'ascorbate de magnésium, l'acide citrique, le citrate de calcium et le citrate de magnésium.

10. Composition diététique selon l'une des revendications 1 à 9, **caractérisée en ce qu'**elle contient en outre 0,01% en poids d'iodure de potassium.

11. Composition diététique sous forme d'un sel de substitution du sel de table, **caractérisée en ce qu'**elle comprend, en poids :
45% de chlorure de potassium
20% de chlorure de sodium
20% de phosphate monocalcique
12% de citrate dibasique de magnésium
1% de carbonate de magnésium
1% d'acide ascorbique
1% d'acide glutamique

12. Utilisation d'une composition selon l'une des revendications 1 à 11 pour la préparation d'une composition diététique pour augmenter les apports en magnésium et en calcium.

13. Utilisation d'une composition telle que définie aux revendications 1 à 11 pour la préparation d'une composition pharmaceutique utile pour le traitement de l'hypertension artérielle légère ou gravidique, la prévention de l'hypertension artérielle, la correction des déficiences en magnésium et/ou le traitement ou la prévention de la rétention hydrosodée.

## Claims

1. Dietetic composition in the form of a salt substitute for table salt, **characterized in that** it comprises, by weight, from:
40% to 50% of potassium chloride
15% to 25% of sodium chloride
15% to 25% of one or more calcium salts
8% to 15% of one or more magnesium salts
and optionally from:
0.5% to 2.5% of one or more antiagglomerating agents
0.5% to 2.5% of one or more taste-enhancing agents.

2. Dietetic composition according to Claim 1, **characterized in that** it comprises, by weight, from:
0.5% to 1% of one or more antiagglomerating agents
0.5% to 2% of one or more taste-enhancing agents.

3. Dietetic composition according to Claim 1 or 2, **characterized in that** it comprises, by weight, from:
45% to 50% of potassium chloride
15% to 20% of sodium chloride
15% to 20% of one or more calcium salts
10% to 15% of one or more magnesium salts
and optionally from:
0.5% to 1% of one or more antiagglomerating agents
0.5% to 2% of one or more taste-enhancing agents.

4. Dietetic composition according to one of Claims 1 to 3, **characterized in that** the calcium salt is monocalcium phosphate, dicalcium phosphate, tricalcium phosphate, calcium glycerophosphate, calcium dicitrate or calcium D-gluconate.

5. Dietetic composition according to one of Claims 1 to 4, **characterized in that** the magnesium salt is a magnesium phosphate, magnesium gluconate or dibasic magnesium citrate.

6. Dietetic composition according to Claim 4, **characterized in that** the calcium salt is monocalcium phosphate.

7. Dietetic composition according to Claim 5, **characterized in that** the magnesium salt is dibasic magnesium citrate.

8. Dietetic composition according to one of Claims 1 to 7, **characterized in that** it comprises, in addition, at least one antiagglomerating agent chosen from magnesium carbonate, colloidal silica, magnesium silicate, stearic acid, magnesium stearate and a calcium phosphate.

9. Dietetic composition according to one of Claims 1 to 8, **characterized in that** it comprises, in addition, at least one taste-enhancing agent chosen from glutamic acid, calcium glutamate, magnesium glutamate, ascorbic acid, calcium ascorbate, magnesium ascorbate, citric acid, calcium citrate and magnesium citrate.

10. Dietetic composition according to one of Claims 1 to 9, **characterized in that** it contains, in addition, 0.01% by weight of potassium iodide.

11. Dietetic composition in the form of a salt substitute for table salt, **characterized in that** it comprises, by weight:
45% of potassium chloride
20% of sodium chloride
20% of monocalcium phosphate
12% of dibasic magnesium citrate
1% of magnesium carbonate
1% of ascorbic acid
1% of glutamic acid.

12. Use of a composition according to one of Claims 1 to 11 for the preparation of a dietetic compoeition for increasing the supply of magnesium and calcium.

13. Use of a composition as defined in Claims 1 to 11 for the preparation of a pharmaceutical composition useful for the treatment of mild or gravidic high blood pressure, the prevention of high blood pressure, the correction of magnesium deficiencies and/or the treatment or prevention of hydrosodium retention.

## Patentansprüche

1. Diätetische Zubereitung in Form eines Ersatzsalzes für Tafelsalz, **dadurch gekennzeichnet, daß** es:
40 bis 50 Gew.-% Kaliumchlorid
15 bis 25 Gew.-% Natriumchlorid
15 bis 25 Gew.-% eines oder mehrerer Calciumsalze
8 bis 15 Gew.-% eines oder mehrerer Magnesiumsalze
und gegebenenfalls:
0,5 bis 2,5 Gew.-% eines oder mehrerer Antiagglomerationsmittel und
0,5 bis 2,5 Gew.-% eines oder mehrerer Geschmacksverbesserer umfaßt.

2. Diätetische Zubereitung nach Anspruch 1, **dadurch gekennzeichnet, daß** sie:
0,5 bis 1 Gew.-% eines oder mehrerer Antiagglomerationsmittel und
0,5 bis 2 Gew.-% eines oder mehrerer Geschmacksverbesserer umfaßt.

3. Diätetische Zubereitung nach Anspruch 1 oder 2, **dadurch gekennzeichnet, daß** sie:
45 bis 50 Gew.-% Kaliumchlorid
15 bis 20 Gew.-% Natriumchlorid
15 bis 20 Gew.-% eines oder mehrerer Calciumsalze
10 bis 15 Gew.-% eines oder mehrerer Magnesiumsalze
und gegebenenfalls:
0,5 bis 1 Gew.-% eines oder mehrerer Antiagglomerationsmittel und
0,5 bis 2 Gew.-% eines oder mehrerer Geschmacksverbesserer umfaßt.

4. Diätetische Zubereitung nach einem der Ansprüche 1 bis 3, **dadurch gekennzeichnet, daß** das Calciumsalz Monocalciumphosphat, Dicalciumphosphat, Tricalciumphosphat, Calciumglycerolphosphat, Calciumdicitrat oder Calcium-Dgluconat ist.

5. Diätetische Zubereitung nach einem der Ansprüche 1 bis 4, **dadurch gekennzeichnet, daß** das Magnesiumsalz ein Magnesiumphosphat, Magnesiumgluconat oder zweibasiges Magnesiumcitrat ist.

6. Diätetische Zubereitung nach Anspruch 4, **dadurch gekennzeichnet, daß** das Calciumsalz Monocalciumphosphat ist.

7. Diätetische Zubereitung nach Anspruch 5, **dadurch gekennzeichnet, daß** das Magnesiumsalz zweibasiges Magnesiumcitrat ist.

8. Diätetische Zubereitung nach einem der Ansprüche 1 bis 7, **dadurch gekennzeichnet, daß** sie zusätzlich mindestens ein Antiagglomerationsmittel ausgewählt aus Magnesiumcarbonat, kolliodalem Siliciumdioxid, Magnesiumsilikat, Stearinsäure, Magnesiumstearat und Calciumphosphaten enthält.

9. Diätetische Zubereitung nach einem der Ansprüche 1 bis 8, **dadurch gekennzeichnet, daß** sie zusätzlich mindestens einen Geschmacksverbesserer ausgewählt aus Glutaminsäure, Calciumglutamat, Magnesiumglutamat, Ascorbinsäure, Calciumascorbat, Magnesiumascorbat, Citronensäure, Calciumcitrat und Magnesiumcitrat enthält.

10. Diätetische Zubereitung nach einem der Ansprüche 1 bis 9, **dadurch gekennzeichnet, daß** sie zusätzlich 0,01 Gew.-% Kaliumjodid enthält.

11. Diätetische Zubereitung in Form eines Ersatzsalzes für Tafelsalz, **dadurch gekennzeichnet, daß** es:
45 Gew.-% Kaliumchlorid
20 Gew.-% Natriumchlorid
20 Gew.-% Monocalciumphosphat
12 Gew.-% zweibasiges Magnesiumcitrat
1 Gew.-% Magnesiumcarbonat
1 Gew.-% Ascorbinsäure und
1 Gew.-% Glutaminsäure
umfaßt.

12. Verwendung einer Zubereitung nach einem der Ansprüche 1 bis 11 für die Herstellung einer diätetischen Zubereitung zur Erhöhung der Magnesium- und Calcium-Zufuhr.

13. Verwendung einer Zubereitung nach einem der Ansprüche 1 bis 11 für die Herstellung einer pharmazeutischen Zubereitung zur Behandlung der leichten oder schweren arteriellen Hypertension, zur Vorbeugung der arteriellen Hypertension, zur Korrektur von Magnesiummangelerscheinungen und/oder zur Behandlung oder zur Vorbeugung der Natriumretention.
